# EUROPEAN PATENT APPLICATION

(11) **EP 0 879 615 A1**
(43) Date of publication of application: **25.11.1998**
(21) Application number: 97108243.3
(22) Date of filing: 21.05.1997
(51) Int. Cl.: A61M 25/09, A61B 5/0215

(54) **Pressure monitoring guide wire**

(71) Applicant: Schneider (Europe) GmbH, 8180 Bülach (CH)
(72) Inventor: Schwager, Michael, 8404 Winterthur (CH)
(74) Representative: Misrachi, Alfred

(57) **Abstract**

The guide wire comprises a shaft 1 with a lumen 4 extending therethrough. The distal area 3 of the shaft has three openings 6 for the entry of pressure waves into the lumen. The openings 6 are distributed along a helical path by 120° shifts in the transverse direction 7 and by shifts corresponding substantially to the length of the openings 6 in the longitudinal direction 8.

## Description

This invention relates to a pressure monitoring guide wire comprising an elongated flexible shaft with proximal and distal portions and a tubular wall defining a lumen therethrough, and a plurality of transverse openings through said tubular wall in the distal portion of the shaft for the entry of pressure waves into the lumen.

The monitoring of fluid pressures during intravascular procedures such as angioplasty, angiography or valvuloplasty, gives a valuable information to the cardiologist to assess both coronary and myocardial flow reserve and collateral blood flow.

Attempts have been to develop hollow guide wires which allow for the measurement of the fluid pressure at the distal end of a catheter from the proximal end thereof. Such guide wires comprise a plurality of side openings in the distal portion of the tubular shaft to allow blood pressure waves to propagate to a pressure sensor connected to the proximal portion of the shaft. The problem of that kind of pressure monitoring guide wires is to provide an uninterrupted lumen throughout the shaft which has to be highly flexible to conform with the tortuous pathways of the blood vessels. Simultaneously, the shaft must have a reasonably high stiffness to assure pushability and torque transmission thereto. And furthermore, the shaft must have a good kink resistance to avoid the risk of constrictions resulting in modification of the advance of pressure waves through the lumen.

Pressure monitoring guide wires have been made of a plastic or metal tube and a stiffening wire. This is however costly and leads to constrictions in the lumen which obstruct the advance of pressure waves through the lumen. Pressure monitoring guide wires have been made of a tube without stiffening wire.

US 5050606 describes a flexible guide wire which can be used for monitoring of fluid pressure during vascular procedures. The guide wire has a main elongated tubular shaft with an inner lumen extending therethrough to an axial port at the distal end thereof. A core member is secured within the inner lumen and extends out of the distal end of the shaft. A tubular extension is disposed about the portion of the core member which extends out of the shaft and is secured at its proximal end to the distal end of the shaft. The distal area of the tubular extension is provided with a plurality of diametrically opposed ports for fluid entry into the tubular extension and shaft. The core has a cross-sectional area substantially less than the smallest transverse cross-sectional area of the lumen of the shaft and tubular extension to whenever possible reduce influence of the core on the passage of fluid pressure pulses. The distal end of the tubular extension is bonded to the core member extending therethrough and also to a helical coil which is disposed about and secured to the portion of the core which extends out of the distal end of the tubular extension. The distal end of the core is secured to a rounded tip at the distal end of the coil.

EP 0313836 A2 shows a pressure monitoring guide wire having a tubular shaft with an axially extending lumen. A tubular member extends axially from the distal end of the shaft in axial alignment with the lumen thereof and with a proximal end received within the distal portion of the shaft. A spring coil ending distally in a tip extends axially from the shaft and surrounds the tubular member. The spring coil, the tubular member and the shaft are soldered together at their connection area. A solid core extends from the distal end of the tubular member within the coil, with its proximal end welded to the tubular member and its distal end welded to the coil by a disk. A safety wire connects the disk and the tip at the distal end of the coil. A plurality of pressure monitoring holes are formed In the wall of the tubular extension, near the distal end thereof and the windings of the coil are spaced apart in the vicinity of the holes to provide unobstructed access thereto. The holes are arranged in three axially spaced rows of holes extending circumferentially around the tubular member and communicating with the lumen of the shaft via the lumen of the tubular member.

EP 0738495 A1 also relates to pressure monitoring guide wires. In one embodiment, the guide wire comprises an elongated flexible shaft with a lumen extending therethrough. The lumen is surrounded by a wall forming the shaft and the distal portion of which is provided with a plurality of diametrically opposed slots for pressure medium entry into the lumen, the slots being arranged in two pairs at a distance from one another. At the distal location of the slots, the wall has a thickness greater than the thickness at the proximal area of the shaft, and a coil surrounds the proximal area of the shaft to compensate the difference in kinking resistance between proximal and distal areas of the shaft. In another embodiment, the guide wire also comprises an elongated flexible shaft with a lumen extending therethrough; the lumen is surrounded by a wall forming the shaft and the distal portion of which is provided with a plurality of diametrically opposed elongated slots located at the same level along the shaft. A coil is located inside the shaft, under the slots for supporting the slotted wall and thereby compensate the difference in kinking resistance between the slotted portion of the shaft and the portion thereof which is devoid of slots.

It is an object of this invention to improve over the cited art by means of a pressure monitoring guide wire which is easy and cheap to manufacture, which is highly versatile while having excellent qualities of pushability and resistance to kinking and which allows a smooth advance of pressure waves through the lumen.

Towards fulfilling of these and other objects the invention complies with definitions given in the claims.

Accordingly, when the plurality of openings is distributed along a path such that the tubular wall is not interrupted by more than one opening if one of either the transverse or longitudinal direction is followed,the weakening effect on the tubular wall resulting from the openings is strongly minimized. The tubular shaft retains the maximal available flexibility at any of the areas provided with an opening. There is no more need for wall supporting cores or coils to compensate a loss of resistance to kinking due to facing openings. There are no transitions or obstructions within the lumen which would interfere on the passage of the fluid pressure pulses. And as the openings do not face each other, there is no generation of turbulences which could trouble the pressure waves signal whereas the plurality of openings may assure a strong detectable signal. And the manufacture of the guide wire is greatly simplified.

The path along which the openings are distributed may be helical for best repartition of flexibility along the apertured portion of the shaft, and the openings may be slots extending longitudinally through the tubular wall for reducing the risk of particles clogging at the entry of the pressure waves.

In a preferred embodiment oriented towards ease of design and manufacture, the openings are distributed along the path by 120° shifts in the transverse direction and by shifts corresponding substantially to a biggest size of the openings in the longitudinal direction.

Where the openings are distributed on a distal portion of the tubular wall having an outer diameter smaller than that of the proximal portion thereof, a further advantage is obtained towards pressure monitoring into tortuous and narrow areas of the vasculature.

These and other objects, features and advantages of the invention will become readily apparent from the following description with reference to the accompanying drawings which show, diagrammatically and by way of example only, a preferred but still illustrative embodiment of the invention.

Figure 1 is a fragmentary centerline sectional view of the tubular shaft of a guide wire according to the invention.

Figures 2, 3 and 4 are cross-sectional views respectively according to lines II-II, III-III and IV-IV of Figure 1.

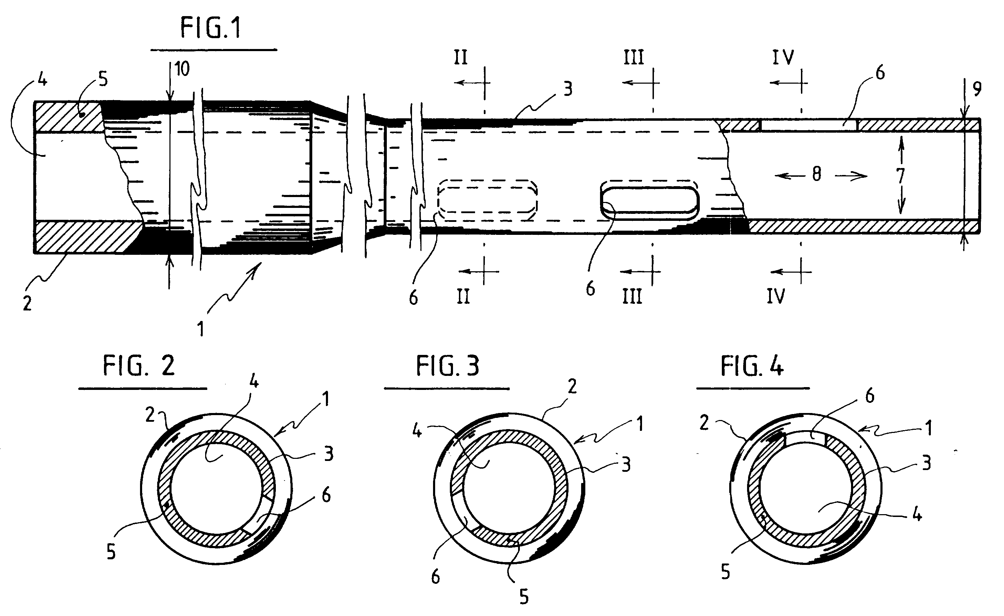

The guide wire shown in Figure 1 comprises an elongated flexible shaft 1 having a proximal area 2 and a distal area 3, the guide wire being shown without a distal tip.

A lumen 4 extends through the shaft 1 and the proximal area 2 of the shaft is intended to be connected to a pressure measuring equipment (not shown) common in the art.

The shaft 1 may be made of stainless steel or of an elastic Nickel Titanium alloy such as for instance Nitinol (Trade Name) or Tinel Alloy (Trade Name). Other materials are also possible, such as for example plastic materials.

The lumen 4 is defined by tubular wall 5 forming the shaft 1.

The distal area 3 of the shaft 1 has three transverse openings 6 formed therein through the wall 5 for the entry of pressure waves into the lumen 4.

The three openings 6, in the form of elongated substantially rectangular slots extending longitudinally through the wall 5, are distributed along a path such that the tubular wall 5 is not interrupted by more than one opening at the same time if one of either the transverse or longitudinal direction 7 and 8 is followed. In the example shown, the three openings 6 are distributed along a helical path by 120° shifts in the transverse direction 7 and by shifts corresponding substantially to the length of the openings 6 in the longitudinal direction 8, as appears from Figures 2, 3 and 4 in conjunction with Figure 1.

In the example shown, the distal area 3 of the shaft 1 has an outer diameter 9 smaller than outer diameter 10 of the proximal area 2, the diameter of lumen 4 being constant.

The distal area 3 of shaft 1 is for termination into a flexible assembly forming a distal tip (not shown), for example as depicted in EP 0738495 A1.

Variants are available without departing from the scope of the invention.

The openings 6 may have a shape other than rectangular, for example a circular or oval shape. And their number may be greater or smaller than three.

Similarly, the openings 6 may be distributed along a path other than helical and their distribution may be other than by 120° shifts in the transverse direction 7 and shifts substantially corresponding to their length in the longitudinal direction 8.

The proximal and distal areas 2 and 3 of shaft 1 may have the same outer diameter.

## Claims

1. A pressure monitoring guide wire comprising an elongated flexible shaft (1) with proximal and distal portions (2, 3) and a tubular wall (5) defining a lumen (4) therethrough, and a plurality of transverse openings (6) through said tubular wall in the distal portion of the shaft for the entry of pressure waves into the lumen, characterized in that the plurality of openings (6) is distributed along a path such that the tubular wall (5) is not interrupted by more than one opening (6) if one of either the transverse or longitudinal direction is followed.

2. A pressure monitoring guide wire according to claim 1, wherein said path is helical.

3. A pressure monitoring guide wire according to claim 1 or 2, wherein said openings (6) are slots extending longitudinally through the tubular wall (5).

4. A pressure monitoring guide wire according to any of claims 1 to 3, wherein the openings (6) are distributed along the path by 120° shifts in the transverse direction (7) and by shifts corresponding substantially to a biggest size of the openings (6) in the longitudinal direction (8).

5. A pressure monitoring guide wire according to any of claims 1 to 4, wherein the openings (6) are distributed on a distal portion (3) of the tubular wall (5) having an outer diameter (9) smaller than that (10) of the proximal portion (2) thereof.
